# EUROPEAN PATENT APPLICATION

(11) **EP 1 002 871 A1**
(43) Date of publication of application: **24.05.2000**
(21) Application number: 98203893.7
(22) Date of filing: 17.11.1998
(51) Int. Cl.: C12P 17/10, C12N 9/02

(54) **Process for preparing optically active 3-hydroxy-pyrrolidine derivatives by enzymatic hydroxylation**

(71) Applicant: Eidgenössische Technische Hochschule (ETH) Zürich, 8092 Zürich (CH)
(72) Inventor: Witholt, Bernard, 8032 Zürich (CH); Li, Zhi, 8003 Zürich (CH)
(74) Representative: Ottevangers, Sietse Ulbe

(57) **Abstract**

The invention provides a process for preparing an optically active 3-hydroxypyrrolidine, wherein an oxygen atom is inserted stereoselectively into the corresponding pyrrolidine by use of a biocatalyst. The biocatalyst may be selected from the group consisting of alkane hydroxylases, alkanes-degrading strains, mono-alicyclic hydrocarbons-degrading strains, microorganisms or enzymes from the genera Pseudomonas, Aspergillus, Mycobacterium, Corynebacterium, Nocardia, Gordona, Beauveria, Fusarium, Bacillus, Streptomyces, Rhizopus, Candida, Rhodococcus, Methylococcus, Sebekia, Penicillium, Mortierella, Gibbeerella, Scopuloriopsis, Curvularia, Gnomonia, Mucor, Wojnowicia, Ophiobolus, Deadalea, Leptosporus, Diaporthe, Colletotrichum, Absidia, Syncephalastrum, Phycomyces, Botryosphaeria, and Nigrospora.

## Description

### Field of the invention

The present invention relates to a process for preparing optically active 3-hydroxypyrrolidine derivatives which are useful as intermediates for the synthesis of several pharmaceutical products and agricultural chemicals, wherein an oxygen atom is inserted stereoselectively into the corresponding pyrrolidine derivatives by use of biocatalysts.

### Description of the Prior Art

Optically active 3-hydroxypyrrolidine and *N*-benzyl 3-hydroxypyrrolidine are useful intermediates for the synthesis of several pharmaceuticals, agrochemicals, and the like.

In practice it is often advantageous, if not required, to use optically active 3-hydroxypyrrolidine in its *N*-protected form.

One process for preparing (*R*)-3-hydroxypyrrolidine involves decarboxylation of (2*S*,4*R*)-4-hydroxy-L-proline [JP 05/255204 (05 Oct. 1993); JP 60/23328 (05 Feb. 1985); Hashimoto, M., et al, *Chem*. *Lett*., **1986**, 893; Mehler, Th., et al, *Synthetic Commun.* **1993**, 23, 2691]. However, the starting material is expensive. (*S*)-3-hydroxypyrrolidine and its *N*-substituted derivatives can be prepared from L-malic acid [EP 692471 (17 Jan. 1996); Bhat, K.L., et al, *Synthetic Commun.*, **1985**, *15*, 587; Naylor, A., et al, *J. Med. Chem*., **1994**, *37*, 2138], L-glutamic acid [Harris, B.D., et al, *Synthetic Commun*., **1986**, *16*, 1815], and L-aspartic acid [Shibata, T. et al, *Heterocycles*, **1986**, *24*, 1331], but all these preparations need multi-step syntheses, and the starting materials are not cheap.

Optically active 3-hydroxypyrrolidine and *N*-substituted derivatives thereof can be prepared by reduction of optically active 4-hydroxy-2-pyrrolidinone derivatives [JP 01/207266 (21 Aug. 1989); JP 01/45360 (17 Feb. 1989)], reduction of optically active 3-hydroxy-2,5-pyrrolidine-dione derivatives [JP 01/254657 (11 Oct. 1989)], cyclization of optically active 4-halo-3-hydroxybutane derivatives [EP 452143 (16 Oct. 91)], and cyclization of optically active 4-halo-3-hydroxy butyl nitrile derivatives [EP 431521 (12 Jun. 1991); JP 03/176463 (31 Jul. 1991); EP 347818 (27 Dec. 1989); EP 269258 (01 Jun. 1988)]. However, the optically active starting materials are not easily available and it needs many steps for their preparation.

Some processes for preparing optically active 3-hydroxypyrrolidine and its *N*-substituted derivatives use resolution of the corresponding racemic compounds by classic methods [JP 05/279326 (26 Oct. 1993); JP 05/279325 (26 Oct. 1993); JP 05/32620 (09 Feb. 1993); JP 04/13659 (17 Jan. 1992); JP 04/164066 (09 Jun. 1992); JP 61/63652 (01 Apr. 1986)], enzymatic hydrolysis [WO 95/03421 (02 Feb. 1995); US 5187094 (16. Feb. 1993); JP 01/141600 (02 Jun. 1989); Hasegawa, J., et al, *Enantiomer*, **1997**, *2*, 311; Tomori, H., et al, *Bull*. *Chem*. *Soc. Jpn.*, **1996**, *69*, 207], and enzymatic esterification [WO 95/03421 (02 Feb. 1995); JP 05/227991 (07 Sep. 1993); JP 04/131093 (01.05.92); Horiguchi, A., et al, *Biosci. Biotech. Biochem*., **1995**, *59*, 1287]. The maximum theoretical yield, however, is 50%, the separation of the product is difficult, and even the preparation of the racemic starting materials needs multi-synthetic steps.

Asymmetric syntheses were also applied in the preparation of optically active 3-hydroxypyrrolidine and *N*-substituted derivatives thereof. Hydroboration of *N*-substituted 3-pyrroline with diisopinocampheylborane followed by oxidation with alkaline hydrogen peroxide gave optically active *N*-substituted 3-hydroxypyrrolidines [Brown, H.C., et al, *J. Am*. *Chem. Soc*., **1986**, *108*, 2049; Brown, H.C., et al, *J. Org. Chem.*, **1986**, *51*, 4296], which is elegant but not suitable for industrial production. Enzymatic reduction of *N*-benzyl-3-pyrrolidinone afforded optically active *N*-benzyl-3-hydroxypyrrolidine [JP 06/141876 (24 May 1994)], but the synthesis of the substrate is difficult.

A more direct and economic method for the preparation of optically active 3-hydroxypyrrolidine and its *N*-substituted derivatives is stereoselective insertion of an oxygen into the corresponding pyrrolidine derivatives which are easily available. However, this type of reaction is very difficult in classic chemistry.

The application of biocatalysts in organic synthesis has developed rapidly and can offer alternative methods to the classic chemical ones. Enzymatic hydroxylation of unactivated cyclic methine group of steroids, for example, can be managed stereoselectively at any desired position by using an appropriate enzyme. Enzymatic hydroxylation of amide-containing bicyclic, polycyclic and spiro compounds was also reported [Holland, H.L. *Organic Synthesis with Oxidative Enzymes,* VCH, **1992**, Ch. 3, p. 55]. However, not much is known about enzymatic hydroxylation of 5-membered mono-heterocyclic hydrocarbons: there is only one report on the hydroxylation of *N*-benzoyl pyrrolidine with *Cunninghamella Verticillate,* which gave the corresponding 3-hydroxylated pyrrolidine in poor yield (Chemical Abstracts, 1993, 118:6835c).

### Summary of the invention

This invention provides a process for the preparation of an optically active 3-hydroxypyrrolidine, wherein an oxygen atom is inserted stereoselectively into the corresponding pyrrolidine by use of a biocatalyst.

More specifically, this invention provides a process for the preparation of an optically active 3-hydroxypyrrolidine derivative having the formula **1** wherein, using a biocatalyst, an oxygen atom is inserted stereoselectively into the corresponding pyrrolidine derivative having the formula **2**: 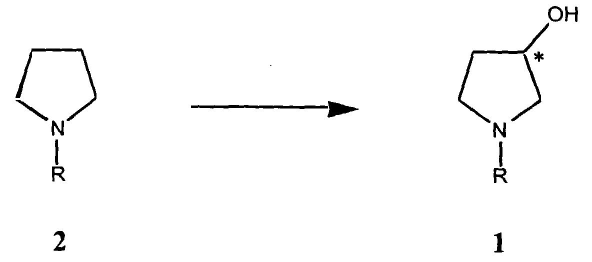 wherein * indicates an asymmetric carbon atom and R represents hydrogen, a linear, cyclic or branched alkyl, alkenyl, aryl, heterocyclic aryl, benzyl, aralkyl, or heterocyclic aralkyl group, containing 1 to 20 carbon atoms, optionally with substitution, or wherein R is C(O)OR², wherein R² represent hydrogen, a linear, cyclic or branched alkyl, alkenyl, aryl, heterocyclic aryl, aralkyl, or heterocyclic aralkyl group, containing 1 to 20 carbon atoms, optionally with substitution, or wherein R is -C(O)R³, R³ represent hydrogen, a linear, cyclic or branched alkyl, alkenyl, aryl except phenyl, heterocyclic aryl, aralkyl, or heterocyclic aralkyl group, containing 1 to 20 carbon atoms, optionally with substitution.

The biocatalysts used are microorganisms or enzymes having hydroxylation activities, or prokaryotic host-organisms having the gene(s) necessary for hydroxylation of pyrrolidines **2** to 3-hydroxy-pyrrolidines **1**.

The biotransformation is performed *in vivo* with resting cells as biocatalysts, *in vivo* with growing cells as biocatalysts, or *in vitro* with crude cell extracts or enzyme preparations that are purified or partially purified as biocatalysts.

The biocatalysts can be immobilized on or in a water-insoluble carrier or support system.

The biotransformation is performed in aqueous medium or in multiphase media possibly containing two or more of the following: a solid phase, an aqueous phase, an organic phase, or a gasiform phase.

The reaction temperature is 5-50°C, preferably at 20-40°C and the pH of the medium is 4-10, preferably 6-8.

The isolation of the optically active 3-hydroxypyrrolidines **1** is performed by separation techniques such as chromatography with an inorganic, organic, or synthetic adsorbent used as a support, by means of extraction, or by membrane filtration.

In a preferred embodiment, *N*-benzyl pyrrolidine was hydroxylated to the corresponding optically active 3-hydroxy *N*-benzyl pyrrolidine, wherein the biocatalyst is *Pseudomonas oleovorans G*po1, *Pseudomonas putida* P1, *Pseudomonas vesicularis*, microorganisms or enzymes having alkane hydroxylase activities, microorganisms or enzymes degrading alkanes or mono-alicyclic hydrocarbons containing 5 or more C atoms, and prokaryotic host organisms having the gene or genes that encode this hydroxylation enzyme system such as the recombinant strain *Escherichia coli* GEc137(pGEc47).

In a preferred embodiment, *N*-tert-butoxycarbonyl pyrrolidine was hydroxylated to optically active 3-hydroxy *N-*tert-butoxycarbonyl pyrrolidine, wherein the biocatalyst is *Aspergillus niger*.

In a preferred embodiment, *N*-acetyl pyrrolidine was hydroxylated to optically active 3-hydroxy *N*-acetyl pyrrolidine, wherein the biocatalyst is one of a series of microorganisms that are capable of degrading cyclohexane.

Optically active *N-*substituted 3-hydroxypyrrolidine obtained by this process can be easily converted into optically active 3-hydroxypyrrolidine by deprotection.

Thus, the invention described herein provides a more direct and economical method for the preparation of optically active 3-hydroxypyrrolidine and *N*-substituted 3-hydroxypyrrolidine by inserting an oxygen atom stereoselectively into the corresponding pyrrolidine derivatives by use of biocatalysts.

### Detailed description of the invention

Here we have developed a process for the preparation of optically active 3-hydroxypyrrolidine derivatives having the formula **1** via stereoselective insertion of an oxygen atom into the corresponding pyrrolidine derivatives having the formula **2** catalysed by microorganisms or enzymes.

In order to find suitable enzymes catalyzing this reaction we screened many microorganisms and enzymes. In a typical screening procedure, a microorganism was inoculated in a nutrient medium suitable for growth, and the culture was incubated with shaking at 25-35°C for 1-3 days. The cells were harvested in the late exponential phase of growth and resuspended in 50 mM phosphate buffer (pH = 7-8). Substrate was added to a concentation of 0.5-10 mM. The mixture was shaken at 25-35°C for 0-3 days. The biotransformation was followed by disappearance of the substrate and appearance of the product. Samples were taken from the reaction mixture and analysed directly by high performance liquid chromatography (HPLC) with a reversed phase column, or the samples were extracted with ethyl acetate and the organic phase was analysed by gas chromatography (GC).

It has been found that microorganisms or enzymes having hydroxylation activities are the suitable biocatalysts. Examples of these biocatalysts are alkane hydroxylases, alkanes-degrading strains, mono-alicyclic hydrocarbons-degrading strains, microorganisms or enzymes from the genera Pseudomonas, Aspergillus, Mycobacterium, Corynebacterium, Nocardia, Gordona, Beauveria, Fusarium, Bacillus, Streptomyces, Rhizopus, Candida, Rhodococcus, Methylococcus, Sebekia, Penicillium, Mortierella, Gibbeerella, Scopuloriopsis, Curvularia, Gnomonia, Mucor, Wojnowicia, Ophiobolus, Deadalea, Leptosporus, Diaporthe, Colletotrichum, Absidia, Syncephalastrum, Phycomyces, Botryosphaeria, and Nigrospora.

It has also been found that the biocatalysts can be prokaryotic host-organisms having gene(s) necessary for hydroxylation of pyrrolidines **2** to 3-hydroxy-pyrrolidines **1**. The recombinant strain *Escherichia coli* GEc137(pGEc47), for example, is a suitable catalyst.

The biotransformation can be performed *in vivo* with resting cells as biocatalysts, *in vivo* with growing cells as biocatalysts, or *in vitro* with purified enzymes or crude cell extracts as biocatalysts.

The biocatalysts can be immobilized on or in a water-insoluble carrier or support system.

The biotransformation can be carried out in aqueous medium. It can also be performed in multiphase media possibly containing two or more of the following: a solid phase, an aqueous phase, an organic phase, or a gasiform phase. Organic solvents with high LogP values can be used as organic phase. This includes alkanes with 5 or more C atoms, dialkyl ethers with 4 or more C atoms, and aromatic hydrocarbons. An example of a suitable organic solvent is hexadecane.

The enzymatic hydroxylations can be carried out, although this is no critical parameter, at a temperature of 5-50°C, preferably at 20-40°C. The pressure can vary within wide limits. In practice the biotransformation is performed at atmospheric pressure. The pH of the reaction medium can be between 4 and 10, preferably between 6 and 8.

The substrates **2** and the corresponding product **1** can be separated easily by chromatographic techniques with an inorganic, organic, or synthetic adsorbent used as a support. The suitable adsorbents are, for instance, aluminium oxide and silicagel.

Hydroxylation of *N*-benzylpyrrolidine to optically active *N*-benzyl-3-hydroxy-pyrrolidine can be catalysed by many microorganisms or enzymes. Suitable biocatalysts are, for example, *Pseudomonas oleovorans G*po1, *Pseudomonas putida* P1, *Pseudomonas vesicularis*, microorganisms or enzymes having alkane hydroxylase activities, microorganisms or enzymes degrading alkanes or mono-alicyclic hydrocarbons containing 5 or more C atoms, and prokaryotic host-organisms having the gene or genes that encode this hydroxylation enzyme system, e.g. the recombinant strain *Escherichia coli* GEc137 (pGEc47).

Hydroxylation with *Pseudomonas oleovorans* Gpo1 (strain ATCC 29347) was demonstrated in examples 1-8, examples 9-11, and example 12, using resting cells, crude cell extracts, and growing cells, respectively. The culture of *Pseudomonas oleovorans* Gpo1 can be prepared either by growing in E2 medium with octane as carbon source described in examples 1-7 or by growing in E2 medium with pyruvate as carbon source followed by induction of the alkane oxidation system with DCPK shown in example 8.

In resting cells experiments 1-8, the *N*-benzyl-3-hydroxypyrrolidine yield increases at higher cell concentrations. It also depended on the concentration of substrate. With a cell concentration of 42 g/L, hydroxylation of *N*-benzylpyrrolidine afforded *N*-benzyl-3-hydroxypyrrolidine in 49% yield starting from 2 mM of substrate and 62% yield from 0.5 mM of substrate, respectively.

In examples 9-11 with crude cell extract, the reaction is quite fast. The yield is dependent on the concentration of the crude cell extract. By use of the crude cell extract obtained from cell densities of 42 g/L, 50% of *N*-benzyl-3-hydroxypyrrolidine was obtained.

In example 12 with growing cells, 12% of *N*-benzyl-3-hydroxypyrrolidine was obtained.

A procedure for following these reactions was established. Samples were taken from the reaction mixture at different time, the cells were removed by centrifugation, and the supernatants were analysed by analytical HPLC.

A procedure for the purification of the products was established. It involved either solvent extraction or chromatography. In the case of chromatography, the unreacted substrates were first eluted from a column of aluminium oxide with hexane/ethyl acetate (1:1), and the product was obtained by washing with methanol. In the case of solvent extraction, *N*-benzylpyrrolidine is first recovered by extraction with an apolar solvent, and optically active *N*-benzyl 3-hydroxypyrrolidine is then extracted out of the remaining reaction mixture after adjusting pH > 12. Suitable extraction agents are alkanes with 5 or more C atoms, dialkyl ethers with 4 or more C atoms, chlorine-containing alkanes with 3 or fewer C atoms, alkyl aromatics with 7-10 C atoms, and carboxylic esters with 3 or more C atoms. Examples of particularly suitable extraction agents are hexane and ethyl acetate.

The pure product was identified as *N*-benzyl-3-hydroxypyrrolidine by comparing the GC-MS and NMR spectra with the corresponding spectra of authentic compound.

The optical purity of *N*-benzyl-3-hydroxypyrrolidine was measured by analytical HPLC with a chiral column [Chiracel OB-H (Daicel), 250 mm x 4.6 mm; eluent: hexane/isopropanol (98:2); flow rate: 0.5 ml/min; detection wavelength: 210 nm; retention times: 26.1 min for the (*R*)-form and 43.5 min for the (*S*)-form]. *N*-benzyl-3-hydroxypyrrolidine obtained from the hydroxylation of *N*-benzylpyrrolidine catalysed by *Pseudomonas oleovorans* Gpo1 has 52% e.e. of the (*R*)-form.

(S)-N-benzyl-3-hydroxypyrrolidine could be prepared from N-benzylpyrrolidine by hydroxylation with *Pseudomonas vesicularis*. In resting cell examples 14-22, best yields were obtained with a cell concentration of 42 g/L: 55%, 44%, and 25% of N-benzyl-3-hydroxypyrrolidine were produced starting from 0.5, 2, and 5 mM of substrate, respectively. The product has 53% e.e. of the (S)-enantiomer. The *Pseudomonas vesicularis* strain used in the examples was isolated from soil samples using n-hexane as carbon source.

Hydroxylation of *N*-benzylpyrrolidine to optically active *N*-benzyl-3-hydroxy-pyrroiidine with *Pseudomonas putida* P1 was shown in example 13 using resting cells. With a cell concentration of 6 g/L, 7% of *N*-benzyl-3-hydroxypyrrolidine was yielded from 2 mM of substrate after 5 h. The product has 64% e.e. of the (*R*)-enantiomer. The *Pseudomonas putida* P1 strain used in the example was isolated from soil samples using n-octane as carbon source.

Use of prokaryotic host-organisms having gene(s) necessary for hydroxylations as biocatalyst is exemplified in example 23. *Escherichia coli* GEc137 (pGEc47), a recombinant strain carrying the genes for a multicomponent alkane hydroxylase from *Pseudomonas oleovorans* Gpo1, catalyses the hydroxylation of *N*-benzylpyrrolidine. 7% of *N*-benzyl-3-hydroxypyrrolidine with 52% e.e. of the (*R*)-enantiomer were obtained. The strain used in the example has been described by G. Eggink et al., J. Biol. Chem. 262, 1987, 17712.

Hydroxylation of *N-*(tert-butoxycarbonyl)-pyrrolidine to optically active 3-hydroxy-*N*-(tert-butoxy-carbonyl)-pyrrolidine was demonstrated in examples 24-25 with *Aspergillus niger* (strain DSM 821) as biocatalyst. 78% of *N*-(tert-butoxy-carbonyl)-3-hydroxy-pyrrolidine with an e.e. of 22% of (*S*)-enantiomer was yielded from the enzymatic hydroxylation of 2 mM of substrate with growing cells. To follow the reactions, samples were taken from the reaction mixture at different times, pH was adjusted to 10, and these samples were then extracted with ethyl acetate. The organic phase was dried and analysed by GC. The separation of substrate and product was achieved by extraction of the reaction mixture with ethyl acetate followed by chromatographic separation on aluminium oxide with hexane/ethyl actate (1:1). The product was identified as *N*-(tert-butoxy-carbonyl)-3-hydroxy-pyrrolidine by comparing the GC-MS and NMR spectra with the corresponding spectra of the authentic compound. E.e of the product was established from the specific rotations.

Hydroxylation of *N*-acetylpyrrolidine to 3-hydroxy-*N*-acetylpyrrolidine was demonstrated in example 26 with cyclohexane degrading strains isolated from soil by classical enrichment techniques. 4% of *N*-acetyl-3-hydroxypyrrolidine was obtained from 2mM of *N*-acetylpyrrolidine by using cells concentation of 8 g/L after 1.5 h. The reaction was followed by analytical HPLC. The product was identified by comparing the GC-MS and NMR spectra with the corresponding spectra of the authentic compound.

### Examples

### Examples 1-8: Preparation of optically active N-benzyl-3-hydroxypyrrolidine in vivo with resting cell of Pseudomonas oleovorans Gpo1

In examples 1-7, *Pseudomonas oleovorans* Gpo1 (strain ATCC 29347) was inoculated in E2 medium with octane vapor as carbon source and grown at 30°C for 10 hours. The cells were harvested at a cell density of 1-2 g/L and resuspended to 4-50 g/L in 50 mM K-phosphate buffer (pH 7.0). *N*-benzylpyrrolidine was added to a final concentration of 0.5-2 mM, and the mixtures were shaken at 30°C for 0-3 days.

In example 8, *Pseudomonas oleovorans* Gpo1 (strain ATCC 29347) was inoculated in E2 medium with 0.4% pyruvate as carbon source at 30°C for 3 hours and then induced with 2mM DCPK for another 3 hours to a cell density of 0.6 g/L. The cells were harvested and resuspended to 6 g/L in 50 mM K-phosphate buffer (pH 7.0). 2 mM of *N*-benzylpyrrolidine was added and the mixture was shaken at 30°C for 0-3 days.

The reaction was followed by analytical HPLC : samples were taken out directly from the reaction mixture at different times, the cells were removed by centrifugation, and the supernatants were analysed by analytical HPLC [column: Spherisorb ODS2 (5 µm), 125 mm x 4 mm; eluent: acetonitrile/10 mM K-phosphate buffer (pH 7.0) 7:3; flow rate: 1 ml/min; detection wavelength: 210 nm; retention time of *N*-benzyl-3-hydroxypyrrolidine: 6.5 min; retention time of *N*-benzylpyrrolidine: 25 min].

The product was isolated according to the following procedure: the reaction mixture was extracted with ethyl acetate to remove the unreacted substrate, the remaining aqueous reaction mixture was adjusted to pH > 12 by the addition of KOH and extracted with ethyl acetate. The organic phase was dried over MgSO₄ and the solvent evaporated. This afforded pure *N*-benzyl 3-hydroxypyrrolidine.

The product can be also isolated as follows: the reaction mixture was adjusted to pH > 12 by the addition of KOH and extracted with ethyl acetate. The organic phase was dried over MgSO₄ and the solvent evaporated. The residue was subjected to chromatography on aluminum oxide with a short column. The unreacted substrate was eluted with hexane/ethyl actate (1:1) first, and then the product was eluted with methanol. The pure product was identified as *N*-benzyl-3-hydroxypyrrolidine by comparing the GC-MS spectra and NMR spectra with the authentic compund. The results of examples 1-8 are listed in table 1.

The optical purity of *N*-benzyl-3-hydroxypyrrolidine was measured by analytical HPLC with a chiral column [Chiracel OB-H (Daicel), 250 mm x 4.6 mm; eluent: hexane/isopropanol (98:2); flow rate: 0.5 ml/min; detection wavelength: 210 nm; retention times: 26.1 min for the (*R*)-form and 43.5 min for the (*S*)-form]. *N*-benzyl-3-hydroxypyrrolidine obtained here has 52% e.e. of the (*R*)-form.

**Table 1**

| Examples No. 1-8, preparation of optically active *N*-benzyl-3-hydroxy-pyrrolidine *in vivo* with resting cells of *Pseudomonas oleovorans* Gpo1 | | | | | | | |
|---|---|---|---|---|---|---|---|
| Ex. No. | Substrate Concentration | Cell concentration | Yield (%) | | | | |
| | | | 1h | 3h | 16h | 23h | 43h |
| 1 | 2 mM | 6 g/L | 5.4 | | | 15.2 | |
| 2 | 2 mM | 9.4 g/L | 2.9 | 5.2 | | 18.6 | |
| 3 | 2 mM | 21 g/L | 8.4 | 18.0 | 23.8 | | 30.7 |
| 4 | 2 mM | 42 g/L | 15.2 | 29.4 | 38.9 | | 49.0 |
| 5 | 0.5 mM | 21 g/L | 11.9 | 23.0 | 29.6 | | 37.8 |
| 6 | 0.5 mM | 42 g/L | 18.4 | 36.1 | 46.5 | | 57.6 |
| 7 | 0.5 mM | 6 g/L | 4.5 | 15.6 | | | |
| 8 | 0.5 mM | 6 g/L | 5.4 | 13.1 | | | |

### Examples 9-11: Preparation of optically active N-benzyl-3-hydroxypyrrolidine in vitro with crude cell extract of Pseudomonas oleovorans Gpo1

In examples 9-11, *Pseudomonas oleovorans* Gpo1 (strain ATCC 29347) was inoculated in E2 medium with octane as carbon source at 30°C with shaking for 10 hours. The cells were harvested and resuspended in Tris-HCl buffer (pH = 7.5) to a concentration of 8-50 g/L. After passage through the French press, the cell debris was removed by centrifugation at 4,000 g. To this crude cell extract containing membrane proteins was added 0.5 mM of *N*-benzylpyrrolidine and 0.5 mM of NADH, and the mixture was shaken at 30°C for 0-3 days. Analytical and isolation procedures were as described above. The results are listed in table 2.

**Table 2**

| Examples No. 9-11, preparation of optically active *N*-benzyl-3-hydroxy-pyrrolidine *in vitro* with crude cell extract of *Pseudomonas oleovorans* Gpo1 | | | | | | | | | |
|---|---|---|---|---|---|---|---|---|---|
| Ex. No. | substrate concentration | crude cell extract from cell con. | NADH (mM) | Yield (%) | | | | | |
| | | | | 0.5h | 1h | 1.5h | 2h | 3h | 4h |
| 9 | 0.5 mM | 9 g/L | 0.5 | 6.8 | 9.8 | 13.7 | 13.5 | 13.1 | 12.7 |
| 10 | 0.5 mM | 21 g/L | 0.5 | 13.1 | 20.6 | 28.8 | 32.3 | 40.4 | 43.9 |
| 11 | 0.5 mM | 42 g/L | 0.5 | 17.2 | 30.0 | 35.6 | 39.4 | 45.8 | 50.2 |

### Example 12: Preparation of optically active N-benzyl-3-hydroxypyrrolidine in vivo with growing cells of Pseudomonas oleovorans Gpo1

*Pseudomonas oleovorans* Gpo1 (strain ATCC 29347) was inoculated in E2 medium with octane vapor as carbon source and grown at 30°C to a cell density of 0.6 g/L. 0.5 mM of *N*-benzylpyrrolidine was added and the cells were allowed to grow further for 3 days. About 12% of *N*-benzyl-3-hydroxypyrrolidine were obtained.

### Example 13: Preparation of optically active N-benzyl-3-hydroxypyrrolidine in vivo with resting cells of Pseudomonas putida P1

*Pseudomonas putida* P1 was inoculated in E2 medium with octane vapor as carbon source and grown at 30°C, the cells were harvested at a cell density of 0.9 g/L and resuspended to 9 g/L in 50 mM K-phosphate buffer (pH 7.0). *N*-benzylpyrrolidine was added to a final concentration of 2 mM, and the mixtures were shaken at 30°C for 0-3 days. Analytical and isolation procedures were as described above. 7% of *N*-benzyl-3-hydroxypyrrolidine was obtained after 5 h. The product has 64% e.e. of the (*R*)-enantiomer.

### Examples 14-22: Preparation of optically active N-benzyl-3-hydroxypyrrolidine in vivo with resting cells of Pseudomonas vesicularis

In examples 14-22, *Pseudomonas vesicularis* was inoculated in E2 medium with octane vapor as carbon source and grown, at 30°C, the cells were harvested at a cell density of 2.5 g/L and resuspended to 9-42 g/L in 50 mM K-phosphate buffer (pH 7.0). N-benzylpyrrolidine was added to a final concentration of 0.5-5 mM, and the mixtures were shaken at 30°C for 0-3 days. Analytical and isolation procedures were as described above. The results are listed in table 3, the product has 53% e.e. of the (*S*)-enantiomer.

**Table 3**

| Examples Nos. 14-22, preparation of optically active N-benzyl-3-hydroxy-pyrrolidine *in vivo* with resting cells of *Pseudomonas vesicularis* | | | | | | | | | |
|---|---|---|---|---|---|---|---|---|---|
| Ex. No. | Substrate Conc. (mM) | Cell Conc. (g/L) | Yield (%) | | | | | | |
| | | | 0.5 h | 1 h | 2 h | 3 h | 5 h | 10 h | 24 h |
| 14 | 5 | 42 | 6.0 | 9.5 | 13.9 | 20.5 | 23.9 | 25.3 | 25.0 |
| 15 | 2 | 42 | 9.0 | 13.0 | 16.9 | 18.6 | 21.2 | 21.7 | 21.2 |
| 16 | 0.5 | 42 | 11.8 | 17.6 | 22.5 | 25.5 | 27.2 | 28.4 | 42.9 |
| 17 | 5 | 21 | 11.5 | 13.1 | 20.3 | 22.9 | 23.9 | 23.2 | 25.0 |
| 18 | 2 | 21 | 18.8 | 30.1 | 33.7 | 40.3 | 41.7 | 42.5 | 44.0 |
| 19 | 0.5 | 21 | 19.3 | 24.7 | 33.4 | 37.9 | 47.0 | 51.4 | 54.7 |
| 20 | 5 | 9 | 7.0 | 10.1 | 12.8 | 13.3 | 14.1 | 14.0 | 15.3 |
| 21 | 2 | 9 | 12.7 | 19.4 | 24.8 | 27.9 | 33.6 | 28.3 | 29.4 |
| 22 | 0.5 | 9 | 19.8 | 28.8 | 45.4 | 50.0 | 53.2 | 47.0 | 54.2 |

### Example 23: Preparation of optically active N-benzyl-3-hydroxypyrrolidine in vivo with resting cells of Escherichia coli GEc137(pGEc47)

*Escherichia coli* GEc137(pGEc47) (described by G. Eggink et al., J. Biol. Chem. 262, 1987, 17712) was inoculated in M9 medium with glucose as carbon source and grown at 37°C for 10 hours to a cell density of 0.2 g/L. Induction was then made by adding 2 mM of DCPK. Cells were harvested at a cell density of 0.6 g/L, and resuspended to 6 g/L in 50 mM K-phosphate buffer (pH 7.0). *N*-benzylpyrrolidine (0.5mM) was added and the mixture was shaken at 30°C for 0-3 days. Analytical and isolation procedures were as described above. 7% of *N*-benzyl-3-hydroxypyrrolidine was obtained after 16 h. The product has 52% e.e. of the (*R*)-enantiomer.

### Examples 24-25: Preparation of optically active N-(tert-butoxycarbonyl)-3-hydroxy-pyrrolidine in vivo with growing cells of Aspergillus niger

*Aspergillus niger* (strain DSM 821) was inoculated at 27°C in a medium containing dextrose (2%), soya peptone (0.5%), yeast extract (0.5%), and K₂HPO₄ (0.5%). The pH was adjusted to 6.5 by adding HCl. After 3 days, *N*-(tert-butoxycarbonyl)-pyrrolidine was added to final concentrations of 2-4 mM and the cells were allowed to grow further for 7 days. To follow the reaction, samples were taken from the reaction mixture at different times, pH was adjusted to 10, samples extracted with ethyl acetate, and the organic phase was dried and analysed by GC [column: Optima 1 (0.25 µm), 30m x 0.32mm; temperature program: 60°C, 2 min, 60-240°C, 25°C/min; detection: FID; retention time of *N*-(tert-butoxycarbonyl)-pyrrolidine: 6.30 min; retention time of *N*-(tert-butoxy-carbonyl)-3-hydroxy-pyrrolidine: 7.61 min]. For work-up, the substrate and product were extracted from the reaction mixture with ethyl acetate followed by chromatographic separation on aluminium oxide with hexane/ethyl acetate (1:1). The pure product was identified as *N*-(tert-butoxycarbonyl)-3-hydroxy-pyrrolidine by comparing the GC-MS spectra and NMR spectra with the authentic compund prepared by chemical synthesis from 3-hydroxy-pyrrolidine and di-*tert*-butyl dicarbonate. [α]_{D}²⁰ = + 5.0 (c=1, CH₂Cl₂), which corresponds to 22% e.e of (*S*)-enantiomer. The results are listed in table 4.

**Table 4**

| Examples Nos. 24-25, preparation of optically active *N*-(tert-butoxycarbonyl)-3-hydroxy-pyrrolidine *in vivo* with growing cells of *Aspergillus niger* | | | | |
|---|---|---|---|---|
| Ex. No. | Substrate Concentration | Yield (%) | | |
| | | 3 days | 4 days | 7 days |
| 24 | 2 mM | 45 | 64 | 78 |
| 25 | 4 mM | | 43 | 52 |

### Example 26: Preparation of N-benzyl-3-hydroxypyrrolidine in vivo with resting cells of cyclohexane-degrading strains

In a resting cell experiment, a mixture of cyclohexane degrading strains (isolated from soil samples with cyclohexane as carbon source) was inoculated in 1/4 of Evans medium without carbon saurce and grown on cyclohexane vapor diluted 10 times by air as carbon source at room temperature for 3 days. The cells were harvested and resuspened to 8 g/L in 50 mM K-phosphate buffer (pH 7.0). *N*-acetylpyrrolidine was added to a concentration of 2 mM, and the mixture was shaken at 30°C for 0-3 days. For following the reaction, samples were taken from the reaction mixture at different times, the cells were removed by centrifugation, and the supernatants were analysed by analytical HPLC [column: Spherisorb ODS2 (5 µm), 125 mm x 4 mm; eluent: acetonitrile/10 mM K-phosphate buffer (pH 7.0) 5/95; flow rate: 1 ml/min; detection wavelength: 210 nm; retention time of *N*-acetyl-3-hydroxypyrrolidine: 2.3 min; retention time of *N*-acetylpyrrolidine: 7.9 min]. 4% of *N*-acetyl-3-hydroxy-pyrrolidine was obtained after 1.5h. The product was identified by comparing the GC-MS spectra and NMR spectra with the authentic compund prepared by chemical synthesis from 3-hydroxy-pyrrolidine and acetyl chloride.

## Claims

1. A process for the preparation of an optically active 3-hydroxypyrrolidine, wherein an oxygen atom is inserted stereoselectively into the corresponding pyrrolidine by use of a biocatalyst.

2. A process according to claim 1 for the preparation of an optically active 3-hydroxypyrrolidine derivative having the formula **1** from the corresponding pyrrolidine derivative having the formula **2**: 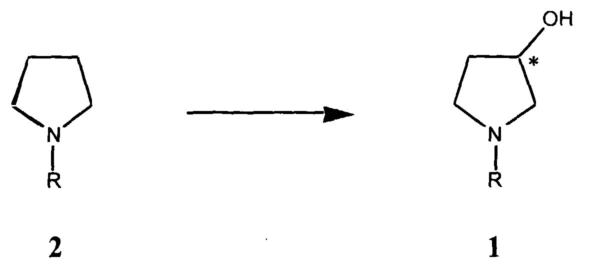 wherein * indicates an asymmetric carbon atom and R represents hydrogen, a linear, cyclic or branched alkyl, alkenyl, aryl, heterocyclic aryl, benzyl, aralkyl, or heterocyclic aralkyl group, containing 1 to 20 carbon atoms, optionally with substitution, or wherein R is C(O)OR², wherein R² represent hydrogen, a linear, cyclic or branched alkyl, alkenyl, aryl, heterocyclic aryl, aralkyl, or heterocyclic aralkyl group, containing 1 to 20 carbon atoms, optionally with substitution, or wherein R is -C(O)R³, R³ represent hydrogen, a linear, cyclic or branched alkyl, alkenyl, aryl except phenyl, heterocyclic aryl, aralkyl, or heterocyclic aralkyl group, containing 1 to 20 carbon atoms, optionally with substitution.

3. A process according to claim 1, wherein the biocatalyst is a microorganism or an enzyme having hydroxylation activity.

4. A process according to claim 1, wherein the biocatalyst is a prokaryotic host-organism having the gene(s) necessary for the hydroxylation of pyrrolidines **2** to 3-hydroxypyrrolidines **1**.

5. A process according to claim 1, wherein the biocatalyst is selected from the group consisting of alkane hydroxylases, alkanes-degrading strains, mono-alicyclic hydrocarbons-degrading strains, microorganisms or enzymes from the genera Pseudomonas, Aspergillus, Mycobacterium, Corynebacterium, Nocardia, Gordona, Beauveria, Fusarium, Bacillus, Streptomyces, Rhizopus, Candida, Rhodococcus, Methylococcus, Sebekia, Penicillium, Mortierella, Gibbeerella, Scopuloriopsis, Curvularia, Gnomonia, Mucor, Wojnowicia, Ophiobolus, Deadalea, Leptosporus, Diaporthe, Colletotrichum, Absidia, Syncephalastrum, Phycomyces, Botryosphaeria, and Nigrospora.

6. A process according to any one of claims 1-5, wherein the biotransformation is performed *in vivo* with resting cells as biocatalyst.

7. A process according to any one of claims 1-5, wherein the biotransformation is performed *in vivo* with growing cells as biocatalyst.

8. A process according to any one of claims 1-5, wherein the biotransformation is performed *in vitro* with crude cell extracts or enzyme preparations that are purified or partially purified as biocatalyst.

9. A process according to any one of claims 1-5, wherein the biocatalyst is immobilized on or in a water-insoluble carrier or support system.

10. A process according to any one of claims 1-9, wherein the biotransformation is performed in aqueous medium.

11. A process according to any one of claims 1-9, wherein the biotransformation is performed in multiphase media containing two or more of the following: a solid phase, an aqueous phase, an organic phase, or a gasiform phase.

12. A process according to claim 11, wherein the organic phase comprises alkanes with 5 or more C atoms, dialkyl ethers with 4 or more C atoms, or aromatic or heteroaromatic hydrocarbons, optionally with substitution.

13. A process according to any one of claims 1-12, wherein the reaction temperature is 5-50°C, preferably 20-40°C.

14. A process according to any one of claims 1-13, wherein the pH of the medium is 4-10, preferably 6-8.

15. A process according to any one of claims 1-14, wherein the optically active 3-hydroxypyrrolidine **1** and the pyrrolidine **2** are separated by column chromatography with an inorganic, organic or synthetic adsorbent used as a support.

16. A process according to any one of claims 1-15, wherein R is a benzyl group.

17. A process according to any one of claims 1-15, wherein R is a *tert*-butoxycarbonyl group.

18. A process according to any one of claims 1-15, wherein R is an acetyl group.

19. A process according to claim 17, wherein the biocatalyst is *Aspergillus niger.*

20. A process according to claim 17, wherein the biocatalyst is a microorganism or enzyme degrading cyclohexane.

21. A process according to claim 16, wherein the biocatalyst is *Pseudomonas oleovorans* Gpo1.

22. A process according to claim 16, wherein the biocatalyst i*s Pseudomonas putida* P1.

23. A process according to claim 16, wherein the biocatalyst is *Pseudomonas vesicularis*.

24. A process according to claim 16, wherein the biocatalyst is a microorganism or enzyme having alkane hydroxylase activity.

25. A process according to claim 16, wherein the biocatalyst is a microorganism or enzyme degrading alkanes or mono-alicyclic hydrocarbons containing 5 or more C atoms.

26. A process according to claim 16, wherein the biocatalyst is a prokaryotic host-organism having a gene coding for an alkane hydroxylase.

27. A process according to claim 26, wherein the biocatalyst is the recombinant strain *Escherichia coli* GEc137(pGEc47).

28. A process according to claim 16, wherein the optically active *N*-benzyl 3-hydroxypyrrolidine and *N*-benzylpyrrolidine are separated by means of extraction, wherein the substrate is first recovered by extraction, and *N*-benzyl 3-hydroxypyrrolidine is then extracted out from the remaining reaction mixture after adjusting pH > 12.

29. A process according to claim 28, wherein the extraction agent used is selected from the group consisting of alkanes with 5 or more C atoms, dialkyl ethers with 4 or more C atoms, chlorine-containing alkanes with 3 or fewer C atoms, alkyl aromatics with 7-10 C atoms, and carboxylic esters with 3 or more C atoms.

30. A process according to any one of claims 1-27, wherein the product and substrate are separated by chromatography on aluminium oxide or silicagel.
